# EUROPEAN PATENT SPECIFICATION

(11) **EP 1 389 137 B1**
(45) Date of publication and mention of the grant of the patent: **19.07.2006**
(21) Application number: 02737094.9
(22) Date of filing: 21.05.2002
(51) Int. Cl.: A61M 1/00, A61M 5/00, A61K 8/00

(54) **COMPOSITIONS FOR PROTEIN DELIVERY VIA THE PULMONARY ROUTE**
ZUSAMMENSETZUNGEN FÜR DIE FREISETZUNG VON PROTEIN AUF DEM PULMONALEN WEG
COMPOSITIONS POUR L'ADMINISTRATION DE PROTEINES PAR LA VOIE PULMONAIRE

(30) Priority: 21.05.2001 US 292783 P
(43) Date of publication of application: 18.02.2004
(73) Proprietor: InJet Digital Aerosols Limited, North Ryde, New South Wales, 2113 (AU)
(72) Inventor: GOODALL, Stephen, Francis, Carindale, QLD 4152 (AU); WATERS, Michael, John, Del Mar, CA 92014 (US); MAHLER, Stephen, Michael, Del Mar, CA 92014 (US); CHAN, Hak-Kim, Del Mar, CA 92014 (US); CHEW, Nora, Y.K., Del Mar, CA 92014 (US)
(74) Representative: Illingworth-Law, William Illingworth
(86) International application number: PCT/US2002/016233
(87) International publication number: WO 2002/094342

(56) References cited:
- EP-A- 0 518 608
- WO-A-92/10990
- WO-A-99/16419
- US-A- 5 413 732
- US-A- 5 894 841
- US-A- 6 028 208
- US-A- 6 095 134
- US-B1- 6 214 873
- US-B1- 6 228 383
- DATABASE WPI Section Ch, Week 198419 Derwent Publications Ltd., London, GB; Class A97, AN 1984-116707 XP002305043 & JP 59 053565 A (DAINIPPON TORYO KK) 28 March 1984 (1984-03-28)

## Description

### FIELD OF INVENTION

The present invention is related to compositions and formulations for the delivery of pharmaceutical compounds via inhalation and, in particular, to formulations for the delivery of proteins and therapeutic agents.

### BACKGROUND

The following description of the background of the invention is provided to aid in understanding the invention, but is not admitted to be prior art to the invention.

Pulmonary drug delivery is an attractive, noninvasive alternative to potentially painful intravenous (IV) injections. Pulmonary drug delivery is particularly useful when oral delivery is not an option, as may often be the case for proteins or peptides, which are readily degraded by enzymes and acid hydrolysis in the gut. Inhalation of substances potentially provides rapid and direct access to the bloodstream via alveoli in the lungs, however, in practice, the efficiency of pulmonary drug delivery depends on at least two factors: the device and its user.

Many therapeutic proteins currently on the market are presently delivered by injection. For example, insulin and insulin derivatives, erythropoetin (EPO), follicle stimulating hormone (FSH), and human growth hormone (hGH), all of which are essentially proteins, are currently administered via injection. In general, any pharmaceutical compound (but especially those currently administered by repetitive injections) is a potential candidate for delivery via the pulmonary route, and might benefit from the advantages disclosed in this invention.

There are currently three primary types of devices used for the delivery of pharmaceuticals to the lungs and each type of these inhalation devices suffers from certain distinct problems, particularly with regard to precise dosing.

One type of device is the metered dose inhaler ("MDI"). MDIs use pressurized gas or propellant to deliver a burst of the compound or pharmaceutical into the patient's mouth during inhalation. The MDI comprises a drug packaged with a propellant in a pressurized aerosol container having a valve which releases a volumetrically metered dose of aerosol upon actuation. These devices are small and portable, but they deliver a dose which may vary undesirably in quantity, delivery speed, and droplet size distribution as the vapor pressure of the propellant varies.

A second type of device is the dry powder inhaler ("DPI"). DPIs use a burst of inspired air to entrain a dose of powder into the bronchial tract. Because the force of inspiration varies from person to person, the administered dose varies from person to person.

A third device type is a nebulizer. Nebulizers generate an aerosol by atomizing a liquid from compressed gas; they deliver an aerosol cloud which contains a pharmaceutical compound. With a nebulizer, as in the MDI device, the dose amount may be regulated by a valve that delivers a metered dose. A mechanical valve actuator may be activated via the patient's inspiration. The dose provided by these devices varies, however, with the vapor pressure of aerosol remaining in the container and the duration of valve actuation.

In general, the poor precision of the above mentioned devices restrict their use to pharmaceutical compounds which have broad dosage tolerance.

In order to deal with these problems, a new type of inhaler has been developed that utilizes resistive actuator technology. This device has been described in U.S. Patent No. 5,894,841. Such an inhalation device may eject a precise amount of an active substance, such as a pharmaceutical compound or formulation into the air path of an inhaler to be entrained into the inhaled air stream of the user. The fact that such an actuator is electronic allows it to be controlled by a microcontroller or microprocessor. This allows inhalers to perform many advanced functions which greatly enhance their performance.

Therefore, there is a need for devices and/or reliable methods and formulations that efficiently, and consistently, deliver precision dosages of a pharmaceutical compound to a patient via the pulmonary route.

### SUMMARY OF THE INVENTION

An embodiment of the present invention comprises a liquid pharmaceutical composition comprising a therapeutic agent, a surface tension-controlling agent, and a component comprising a humectant and a viscosity-controlling agent.

Another embodiment of the present invention comprises a device for pulmonary delivery of a therapeutic protein to a patient, the device comprising a computer-controlled electronic aerosol generating system fluidly connected to a reservoir containing a liquid pharmaceutical composition comprising a therapeutic protein, a surface tension-controlling agent and a humectant.

Another embodiment of the present invention comprises a method of making a device for pulmonary delivery of a therapeutic protein to a patient, the method comprising fluidly connecting a computer-controlled electronic aerosol generating system to a reservoir containing a liquid pharmaceutical composition comprising a therapeutic protein, a surface tension-controlling agent and humectant.

Embodiments of the present invention described herein use the proteins insulin, follicle stimulating hormone (FSH), and human growth hormone (hGH) as therapeutic agents. Other suitable protein growth hormones include human granulocyte colony stimulating factor (G-CSF), granulocyte macrophage colony stimulating factor (GM-CSF), macrophage colony stimulating factor (M-CSF), colony stimulating factor (CSF), and leuteinizing hormone (LH).

Other suitable therapeutic proteins used in accordance with embodiments of the present invention comprise hematopoietic growth factor, interleukins, interferons, cell adhesion proteins, angiogenic proteins, blood coagulation proteins, thrombolytic proteins, bone morphogenic proteins, glucagon and glucagon-like proteins, hormones, receptors, antibodies, and enzymes.

An embodiment of the present invention improves thermal droplet ejection device performance by optimizing: (1) kogation and crusting, (2) fluid flow, droplet size and droplet formation, and (3) and maintenance of therapeutically relevant protein concentrations and maintenance of protein integrity before, during, and after formulation aerosolation.

Advantageously, an embodiment of the present invention provides uniform size and composition of droplets used in a droplet ejection device.

Further features and advantages of the invention as well as the operation of various embodiments of the invention are described in detail herein with reference to the accompanying drawings.

### BRIEF DESCRIPTION OF THE DRAWINGS

The present invention will be described with particular embodiments thereof, and references will be made to the drawings in which:
Figure 1 is a bioassay comparison of human growth hormone formulation before and after aerosolization according to an embodiment of the present invention; and
Figure 2 is a reverse phase HPLC Analysis of Insulin before (upper graph), after initial aerosolization (middle graph) and after repeated aerosolization (lower graph) according to an embodiment of the present invention.

### DETAILED DESCRIPTION OF AN ILLUSTRATIVE EMBODIMENT

For convenience in the ensuing description, the following explanations of terms are adopted. However, these explanations are intended to be exemplary only. They are not intended to limit the terms as they are described or referred to throughout the specification. Rather these explanations are meant to include any additional aspects and/or examples of the terms as described and claimed herein.

The term "pharmaceutical compound", or "therapeutic agent," shall be interpreted to mean any molecule or mixture of molecules which provides a therapeutic, prophylactic, or diagnostic effect.

The terms "peptide" and "protein" are interchangeable, and as described herein shall mean oligopeptides, proteins and recombinant proteins and conjugates thereof, especially those identified as having therapeutic or diagnostic potential. Non-naturally occurring proteins and peptides conjugated to non-protein therapeutic compounds also fall within the scope of these terms.

According to an embodiment of the present invention, a thermal droplet ejection device comprises a dispensing substance container fluidly connected to an array of fluid chambers and each has a resistor, each resistor being located behind a nozzle. Each nozzle ejects a droplet(s) of liquid from the chamber if and when the corresponding resistor is energized by an electrical pulse. Within a fraction of a second, liquid in contact with the resistor is vaporized and forms a bubble. The vapor bubble grows rapidly and imparts momentum to liquid above the bubble and some of this liquid is ejected as a droplet from the adjacent nozzle. The volume of ejected liquid is automatically replaced in the fluid chamber from a container by capillary action or by atmospheric pressure acting on a collapsible bladder, or by a piston or the like. A piezoelectric device generates a droplet by means of a pressure wave in the fluid produced by applying a voltage pulse to a piezoelectric ceramic channel which in this device acts as the ejection means. As with the thermal device, the droplet is ejected through a nozzle. The fluid is ejected in the form of a droplet(s) whose velocity depends on the energy contained in the applied pulse.

Modern printing processes, such as an ink jet printing device, may also use heat energy from a resistor to vaporize a thin layer of ink at the bottom of a well, forming an expanding vapor-phase bubble near the jet. The bubble of vapor, sometimes called the driver, forces ink through a nozzle and out towards the paper. Thermal ink jet cartridges are configured to fire droplets at frequencies of greater than 10 kilohertz and the configuration of a resistor, inkwell and nozzle may be replicated many hundreds of times in closely spaced and intricate patterns to provide greater printing efficiency.

Droplet ejection devices and ink jet printing devices differ in several ways. For example, the nozzle arrays in printing devices are engineered to deposit droplets on a plane of paper whereas inhalation devices are engineered to deposit formula-containing droplets deep into at least one lung of the patient. In general, droplet ejection devices for use in an inhalation device may have a smaller and/or greater number of nozzles, resistors and fluid chambers than those used for printing. Further, there is no need for the nozzles to be arranged in a rectangular matrix with parallel nozzle axes as often the case in ink jets for printing. The droplet ejection nozzles may, for example, be arranged in a circle and/or may be directed at a converging or diverging angle to the axis of each other.

In some cases it is desirable to eject much smaller droplets than are useful for printing ink jets; the construction of the nozzles, resistors and chambers may therefore differ in size and construction from those employed in ink jet printing to produce the required smaller droplets. Additionally, droplet ejection nozzles may differ in diameter such that the particle size of the active agent sprayed from the device may be controlled programmatically by selecting which nozzles are used for droplet ejection; particle size may be varied from one time interval to another.

Aerosols are suspended particles of solid or droplets of liquid in a gaseous medium, such as air drawn through a device by the user. Providing medicaments, in accordance with an embodiment of the present invention, as an aerosolized solution ejected from an ink jet creates a plume of small droplets that can be inhaled; the inhaled droplets are then deposited in the alveoli of the lungs. It is therefore useful for the composition of droplets to maintain the composition of the solution in the reservoir. This prevents formulation components, for example, a solvent, from partitioning unequally between the droplets ejected and the solution remaining in the device between uses. Such partitioning could for example lead to a substantial solute concentration (and eventually precipitation and kogation) or alternatively, a depletion of one or more medicament between the material ejected and the material remaining in the device. Such undesirable partitioning could affect the dose by altering an actual dose from an intended dose.

Another dosage-effecting factor is the size of the droplets. Droplet size is in turn affected by the composition of the aerosolized medicament and how these components behave under the conditions of ink jet bubble delivery. The compositions of such formulations are the topic of the present disclosure.

Droplet ejection devices subject the materials to certain physical forces, for example heat, sheering forces, and surface tension. These forces may affect more sensitive components of the formulation, for example, by causing the components to impinge precise and controlled dosing method. Proteins are labile and will deform and denature with elevated temperatures. The heat incurred by the resistor is to some extent transferred to the liquid and ejected droplets. Localized heating which is not effectively dissipated may deleteriously affect sensitive components of any formulation. It is thus useful both to minimize, and to account for, such effects, as done in an embodiment of the present invention.

As used in an embodiment of the present invention, surface tension and viscosity agents facilitate the flow of liquid through the device channels. Surface tension and viscosity affect the reliable formation of correctly sized droplets by assisting the ejected liquid bolus to coalesce into uniform-sized droplets after release from the nozzle. Correct droplet size affects the correct and controlled dosage levels, especially for inhalers that deliver a predetermined numbers of droplets.

The molecules at the surface of a liquid are subject to strong attractive forces of the interior molecules. A resultant force, whose direction is in a plane tangent to the surface at a particular point, acts to make the liquid surface as small as possible. The magnitude of this force is called surface tension and has the units of force per unit length (surface tension is in dynes per centimeter). Surface tension in the range of 8 dynes/cm to 75 dynes/cm is used in an embodiment of the present invention. Alternatively, the surface tension is between 10 dynes/cm and 50 dynes/cm. Alternatively still, it is between 25 dynes/cm and 50 dynes/cm. Alternatively still, it is between 25 dynes/cm and 35 dynes/cm. Alternatively still, the surface tension of the formulation may be adjusted to any value as needed.

Surface tension is a factor which affects droplet size and may be controlled by the introduction of surface tension-controlling agents, such as surfactants. Surfactants that are charged or non ionic agents are used in an embodiment of the present invention. The amount of surfactant used in a particular formulation depends on the nature of surfactant and in particular, the molecular weight of that agent. Liquid compositions wherein the surface tension-controlling agent comprises 0.01% to 3% w/v of composition are used according to an embodiment of the present invention. Alternatively, the surface tension-controlling agent comprises 0.05% to 0.15% w/v of composition. Alternatively, the surface tension-controlling agent can be adjusted to any w/v of the composition, as needed.

Viscosity is a sort of internal friction and is a measure of a liquid's resistance to changes in shape. Viscosity effects come into play if the liquid formulation in the present invention changes its shape as it moves, such as when the formulation is forced through a nozzle. Such viscosity effects may be controlled by agents such as polyethylene glycols, alcohols and the like. Viscosity is temperature dependent and may be expressed in units of centipoise (cp). Formulations having viscosities in the range of 1 cp to 10 cp at 25 °C are used in an embodiment of the present invention. Alternatively, the amount of viscosity-controlling agent may be adjusted as needed.

Kinematic viscosity is an alternative expression of viscosity defined as the viscosity of a fluid divided by its density. Liquid formulations of the present invention have densities ranging from of 0.7 g/mL to 2.2 g/mL. Alternatively, the densities range from 0.5 g/mL to 3.0 g/mL. Alternatively still, the densities can vary as needed.

According to an embodiment of the present invention, it is useful to ensure that the composition of the aerosol emanating from the device is the same in all discernable aspects as the formulation filled into the device. For example, it is useful to ensure that changes in component concentration and/or in bioactivity occur in a controlled manner.

The well from which the aerosol is ejected is small, thus the total volume of formulation is also small. The action of vaporizing the driver may, over time, leave an accumulation of debris on the resistor surface, an effect known as kogation. Kogation also tends to clog the jet, causing the jets to sometimes fail or to sometimes produce an erratic delivery of dislodged particles from the resistor surface to the aerosol. The addition of humectants (hydrophilic agents) counteracts kogation.

Humectants such as polyethylene glycol (PEG) and cyclodextrin are used in an embodiment of the present invention. In another embodiment, the humectant also comprises a viscosity-controlling agent, because formulations employing higher molecular weight PEGs are more viscous. PEG molecular weights ranging from 600 to 8000 daltons can be used. Alternatively, other commercially available PEGs or related molecules are used as humectants.

Successful additives are largely inert towards pharmaceutical components, e.g., interact only non-specifically with proteins without forming covalent bonds which could change the tertiary structure of the protein. On the other hand, protein tertiary structure stability is known to correlate with the number of hydrogen bonds and ionic interactions between charged groups. For this reason, the addition of humectants as salts might therefore lead to decreased protein stability. Humectants which are nonionic may have different effects than ionic ones. While the addition of organic solvents might be thought to encourage hydrogen bonding and therefore stabilize protein tertiary structure, such solvents might lead to protein insolubility, thus defeating the aim of obtaining uniform formulation delivery.

Accordingly, the development of formulations suitable for protein drug delivery or thermal droplet ejection drug dosing benefits by an embodiment of the present invention that resolves one or more of the following issues: (1) kogation and crusting, (2) fluid flow and droplet formation, and (3) protein protection as described above. In addition, the embodiment provides formulations that are compatible with particular proteins or pharmaceutical agents.

Therefore, an embodiment of the present invention comprises a liquid pharmaceutical composition comprising a therapeutic agent, a surface tension-controlling agent, and a component comprising a humectant and a viscosity-controlling agent.

### EXAMPLES

Examples of embodiments of the present invention are exemplified in the following examples. These examples are merely for illustrative purposes only and are not meant to be limiting on the scope of the appended claims.

The surface tension of the solution was estimated using a strain gauge tensiometer referenced against pure water. Commercially available surfactants such as TWEEN® 20 (ICI Americas Inc.) (polyoxyethylene(20) sorbitan monolaurate) TWEEN® 80, (polyoxyethylene (20) sorbitan monooleate), cetrimide (alkyltrimethylammonium bromide), and BRIJ® 35 (ICI Americas Inc.) (polyoxoethylene(23) lauryl ether) were used. Other surfactants related to those tested are also candidate surfactants.

Proteins were tested with a transgenic cell bioassays with receptors introduced to the cells specific to the test protein. These bioassays were appropriate for indicating the functional effectiveness of the protein. Molecular assays were performed to indicate the primary or tertiary structure of the molecule. Assays used included size exclusion high performance liquid chromatography, reverse phase high performance liquid chromatography, mass spectrometry, isoelectric focusing and immunoassay.

Formulations were developed that would exhibit no kogation and the bioactivity and molecular structure of the protein was unaffected by the process of aerosolizing the formulation and recovering the condensed aerosol. Each protein was formulated at a concentration that was calculated to be of clinical significance such that the plume of aerosol contained the required amount of therapeutic. Humectant was added to the protein formulation which was filled into an inkjet cartridge and fired from the cartridge.

Humectant and surfactant concentrations were selected such that the dose ejected from the device did not diminish with repeated firings and the ejected aerosol cloud was consistent.

Resistor surfaces and nozzles were inspected by Scanning Electon Microscopy to establish the cleanliness of the surfaces and subsequently the effectiveness of the humectant and surfactant. When humectant and surfactant concentrations were suitable, the aerosol from the device was collected, analyzed, and compared with the solution before aerosolization.

During testing, it was useful to ensure that the composition of the aerosol emanating from the device was the same in all discernable aspects as the formulation filled into the device. For example, it was useful to ensure that changes in component concentration and/or in bioactivity occurred in a controlled manner.

### EXAMPLE 1

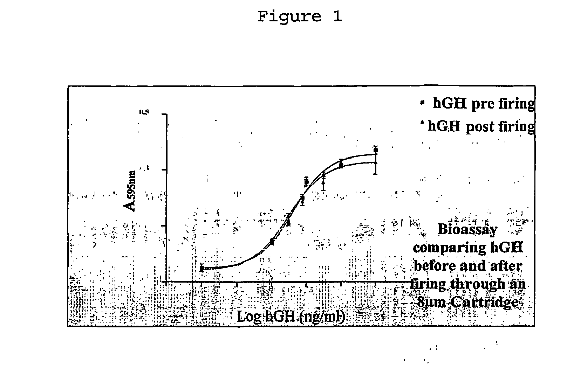

Human growth hormone (hGH) was formulated at 2 mg/mL with 6% w/w PEG 8000 and 0.1% w/v TWEEN® 20. The formulation was unbuffered and no additional salts were added to the formulation. This formulation was aerosolized using a thermal inkjet and the aerosol condensed and recovered for analysis by bioassay and HPLC. The formulation aerosol had an average droplet size of 8 µm as measured by a Malvern laser. An example of the bioassay result for the recovered aerosol according to an embodiment of the present invention is shown in Figure 1. These analyses indicated there was no measurable difference between the two solutions, which established the protein (and thus the formulation) was not materially affected during or after dosage.

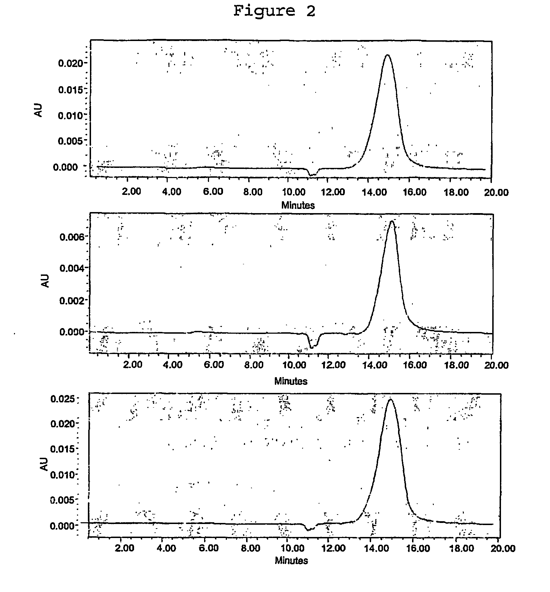

Insulin was formulated at 10 mg/mL with 6% w/v PEG 8000 and 0.1% w/v TWEEN® 80 and aerosolized using thermal inkjet. The aerosol was recovered and compared with the initial formulation using a cell bioassay and reverse-phase HPLC. Figure 2 is a representative HPLC trace of insulin before aerosolization (the upper graph), after recovered aerosolization (the middle graph), and after repeated aerosolization (the lower graph), according to an embodiment of the present invention. Identical retention time and peak shape for all three solutions indicated that there was no measurable differences between the solutions, establishing protein (and thus formulation) integrity during and after dosage.

### EXAMPLE 2

Similar tests were carried out on follicle stimulating hormone (FSH) formulated at 150 ng/mL. Mass spectrometry, HPLC, isoelectric focussing and immunoassay tests performed on the recovered aerosol and initial formulation indicated there was no difference between the solutions during or after dosage.

### EXAMPLE 3

Other non-protein therapeutic agents, nicotine, cromolyn sodium, and nedocromil, were formulated and aerosolized using procedures similar to those used for protein formulations. Nicotine was formulated at concentrations up to 50% w/w in water with 0.1% w/v TWEEN® 20 and aerosolized using this procedure. According to these tests there was no measurable difference between the solution during or after dosage.

Other excipients and solutes may be used in conjunction with this technology. Such excipients include alcohols, hydrocarbons and fluorocarbons, which may also advantageously enhance or control formulation viscosity and also drug solubilties. Furthermore, it has been found that it is not necessary for the drug to be in solution, it also may be present as an emulsion or suspension.

## Claims

1. A liquid pharmaceutical composition suitable for aerosolisation by a thermal droplet ejection device so as to be administered via inhalation, said composition comprising a therapeutic agent, a surface tension-controlling agent, and a component comprising a humectant and a viscosity-controlling agent, and wherein the composition has a surface tension of 8 dynes/cm to 75 dynes/cm.

2. A liquid composition according to claim 1 that is an aqueous solution.

3. A liquid composition according to claim 1 wherein the therapeutic agent is a protein selected from the group consisting of a hormone, a receptor, an antibody, and an enzyme.

4. A liquid composition according to claim 1 wherein the therapeutic agent is a protein selected from the group consisting of a hematopoietic growth factor, an interleukin, an interferon, a growth hormone, a cell adhesion protein, an angiogenic protein, a coagulation protein, a thrombolytic protein, a bone morphogenic protein.

5. A liquid composition according to claim 1 wherein the therapeutic agent is a hematopoietic growth factor selected from the group consisting of EPO, G-CSF, GM-CSF, M-CSF, and SCF.

6. A liquid composition according to claim 1 wherein the therapeutic agent is a hormone selected from the group consisting of insulin, glucagon, growth hormone, FSH, and LH.

7. A liquid composition according to claim 1 wherein the therapeutic agent is an antibody or an antibody fragment conjugated to a therapeutic compound.

8. A liquid composition according to claim 1 wherein the therapeutic agent is a recombinant protein.

9. A liquid composition according to claim 1 wherein the therapeutic agent is a non-naturally occurring protein.

10. A liquid composition according to any preceding claim wherein the surface tension-controlling agent is a surfactant.

11. A liquid composition according to claim 10 wherein the surfactant is selected from the group consisting of an anionic, non ionic, a zwitterionic and cationic agents.

12. A liquid composition according to any preceding claim wherein the surface tension-controlling agent comprises 0.01% to 3% w/v of the composition.

13. A liquid composition according to any preceding claim wherein the viscosity-controlling agent is a polymer.

14. A liquid composition according to any preceding claim wherein the viscosity-controlling agent is a polyethyleneglycol.

15. A liquid composition according to claim 14 wherein the polyethyleneglycol has a molecular weight of 1 to 20 kilodaltons.

16. A liquid composition according to any preceding claim wherein the viscosity-controlling agent provides for a solution having a viscosity of 2 cp to 10 cp.

17. A liquid composition according to any preceding claim wherein the component comprising the viscosity-controlling agent and the humectant is a PEG.

18. A liquid composition according to any preceding claim having a density of 0.7 g/mL to 2.2 g/mL.

19. A fluid reservoir comprising a reservoir containing a liquid composition according to any preceding claim.

20. Use of a thermal droplet ejection device to produce an aerosol from a liquid composition according to any one of claims 1 to 18.

21. A device for pulmonary delivery of a therapeutic protein to a patient, the device comprising a computer-controlled electronic aerosol generating system fluidly connected to a reservoir containing a liquid pharmaceutical composition comprising a therapeutic protein, a surface tension-controlling agent and a humectant, and wherein the composition has a surface tension of 8 dynes/cm to 75 dynes/cm.

22. The device according to claim 21 wherein the humectant comprises a viscosity-controlling agent.

23. A method for making a device for pulmonary delivery of a therapeutic protein to a patient, the method comprising fluidly connecting a computer-controlled electronic aerosol generating system to a reservoir containing a liquid pharmaceutical composition comprising a therapeutic protein, a surface tension-controlling agent and a humectant, and wherein the composition has a surface tension of 8 dynes/cm to 75 dynes/cm.

24. The method according to claim 23 wherein the humectant comprises a viscosity-controlling agent.

## Patentansprüche

1. Flüssige pharmazeutische Zusammensetzung, die für eine Aerosolbildung durch eine thermische Tröpfchenausstoßvorrichtung geeignet ist, so daß sie durch Inhalation verabreichbar ist, wobei die Zusammensetzung ein therapeutisches Mittel, ein die Oberflächenspannung steuerndes Mittel und eine Komponente, die ein Netzmittel und ein die Viskosität steuerndes Mittel umfaßt, enthält und wobei die Zusammensetzung eine Oberflächenspannung von 8 Dyn/cm bis 75 Dyn/cm hat.

2. Flüssige Zusammensetzung nach Anspruch 1, welche eine wäßrige Lösung ist.

3. Flüssige Zusammensetzung nach Anspruch 1, wobei das therapeutische Mittel ein Protein ist, das aus der Gruppe ausgewählt ist, bestehend aus einem Hormon, einem Rezeptor, einem Antikörper und einem Enzym.

4. Flüssige Zusammensetzung nach Anspruch 1, wobei das therapeutische Mittel ein Protein ist, das aus der Gruppe ausgewählt ist, bestehend aus einem hämatopoetischen Wachstumsfaktor, einem Interleukin, einem Interferon, einem Wachstumshormon, einem Zelladhäsionsprotein, einem angiogenen Protein, einem Koagulationsprotein, einem thrombolytischen Protein und einem knochenbildenden Protein.

5. Flüssige Zusammensetzung nach Anspruch 1, wobei das therapeutische Mittel ein hämatopoetischer Wachstumsfaktor ist, der aus der Gruppe ausgewählt ist, bestehend aus EPO, G-CSF, GM-CSF, M-CSF und SCF.

6. Flüssige Zusammensetzung nach Anspruch 1, wobei das therapeutische Mittel ein Hormon ist, das aus der Gruppe ausgewählt ist, bestehend aus Insulin, Glukagon, Wachstumshormon, FSH und LH.

7. Flüssige Zusammensetzung nach Anspruch 1, wobei das therapeutische Mittel ein Antikörper oder ein Antikörperfragment ist, der/das an eine therapeutische Verbindung konjugiert ist.

8. Flüssige Zusammensetzung nach Anspruch 1, wobei das therapeutische Mittel ein rekombinantes Protein ist.

9. Flüssige Zusammensetzung nach Anspruch 1, wobei das therapeutische Mittel ein nicht natürlich vorkommendes Protein ist.

10. Flüssige Zusammensetzung nach einem der vorangegangenen Ansprüche, wobei das die Oberflächenspannung steuernde Mittel ein Tensid ist.

11. Flüssige Zusammensetzung nach Anspruch 10, wobei das Tensid aus der Gruppe ausgewählt ist, bestehend aus anionischen, nicht-ionischen, zwitterionischen und kationischen Mitteln.

12. Flüssige Zusammensetzung nach einem der vorangegangenen Ansprüche, wobei das die Oberflächenspannung steuernde Mittel 0,01 % bis 3 % w/v der Zusammensetzung ausmacht.

13. Flüssige Zusammensetzung nach einem der vorangegangenen Ansprüche, wobei das die Viskosität steuernde Mittel ein Polymer ist.

14. Flüssige Zusammensetzung nach einem der vorangegangenen Ansprüche, wobei das die Viskosität steuernde Mittel ein Polyethylenglycol ist.

15. Flüssige Zusammensetzung nach Anspruch 14, wobei das Polyethylenglycol ein Molekulargewicht von 1 bis 20 Kilodalton hat.

16. Flüssige Zusammensetzung nach einem der vorangegangenen Ansprüche, wobei das die Viskosität steuernde Mittel eine Lösung mit einer Viskosität von 2 cP bis 10 cP liefert.

17. Flüssige Zusammensetzung nach einem der vorangegangenen Ansprüche, wobei die Komponente, welche das die Viskosität steuernde Mittel und das Netzmittel umfaßt, ein PEG ist.

18. Flüssige Zusammensetzung nach einem der vorangegangenen Ansprüche mit einer Dichte von 0,7 g/ml bis 2,2 g/ml.

19. Fluidreservoir, das ein Reservoir umfaßt, welches eine flüssige Zusammensetzung nach einem der vorangegangenen Ansprüche enthält.

20. Verwendung einer thermischen Tröpfchenausstoßvorrichtung zur Erzeugung eines Aerosols aus einer flüssigen Zusammensetzung nach einem der Ansprüche 1 bis 18.

21. Vorrichtung für die pulmonare Auslieferung eines therapeutischen Proteins an einen Patienten, wobei die Vorrichtung ein computergesteuertes, elektronisches, Aerosol erzeugendes System umfaßt, das in einer Fluidverbindung mit einem Reservoir steht, welches eine flüssige pharmazeutische Zusammensetzung enthält, die ein therapeutisches Protein, ein die Oberflächenspannung steuerndes Mittel und ein Netzmittel umfaßt, und wobei die Zusammensetzung eine Oberflächenspannung von 8 Dyn/cm bis 75 Dyn/cm hat.

22. Vorrichtung nach Anspruch 21, wobei das Netzmittel ein die Viskosität steuerndes Mittel umfaßt.

23. Verfahren zur Herstellung einer Vorrichtung für die pulmonare Auslieferung eines therapeutischen Proteins an einen Patienten, wobei das Verfahren die Fluidverbindung eines computergesteuerten, elektronischen, Aerosol erzeugenden Systems mit einem Reservoir umfaßt, welches eine flüssige pharmazeutische Zusammensetzung enthält, die ein therapeutisches Protein, ein die Oberflächenspannung steuerndes Mittel und ein Netzmittel umfaßt,_ und wobei die Zusammensetzung eine Oberflächenspannung von 8 Dyn/cm bis 75 Dyn/cm hat.

24. Verfahren nach Anspruch 23, wobei das Netzmittel ein die Viskosität steuerndes Mittel umfaßt.

## Revendications

1. Composition pharmaceutique liquide apte à la transformation en aérosol par un dispositif thermique d'éjection de gouttelettes de manière à permettre son administration par inhalation, ladite composition comprenant un agent thérapeutique, un agent d'ajustement de tension superficielle et un constituant comprenant un agent hydratant et un agent de régulation de viscosité, composition qui possède une tension superficielle de 8 dynes/cm à 75 dynes/cm.

2. Composition liquide suivant la revendication 1, qui est une solution aqueuse.

3. Composition liquide suivant la revendication 1, dans laquelle l'agent thérapeutique est une protéine choisie dans le groupe consistant en une hormone, un récepteur, un anticorps et une enzyme.

4. Composition liquide suivant la revendication 1, dans laquelle l'agent thérapeutique est une protéine choisie dans le groupe consistant en un facteur de croissance hématopoïétique, une interleukine, un interféron, une hormone de croissance et une protéine d'adhésion cellulaire, une protéine angiogénique, une protéine de coagulation, une protéine thrombolytique et une protéine morphogène osseuse.

5. Composition liquide suivant la revendication 1, dans laquelle l'agent thérapeutique est un facteur de croissance hématopoïétique choisi dans le groupe consistant en EPO, G-CSF, GM-SCF, M-CSF et SCF.

6. Composition liquide suivant la revendication 1, dans laquelle l'agent thérapeutique est une hormone choisie dans le groupe consistant en insuline, glucagon, hormone de croissance, FSH et LH.

7. Composition liquide suivant la revendication 1, dans laquelle l'agent thérapeutique est un anticorps ou un fragment d'anticorps conjugué à un composé thérapeutique.

8. Composition liquide suivant la revendication 1, dans laquelle l'agent thérapeutique est une protéine recombinante.

9. Composition liquide suivant la revendication 1, dans laquelle l'agent thérapeutique est une protéine non naturelle.

10. Composition liquide suivant l'une quelconque des revendications précédentes, dans laquelle l'agent d'ajustement de tension superficielle est un agent tensioactif.

11. Composition liquide suivant la revendication 10, dans laquelle l'agent tensioactif est choisi dans le groupe consistant en un agent anionique, un agent non ionique, un agent zwitterionique et un agent cationique.

12. Composition liquide suivant l'une quelconque des revendications précédentes, dans laquelle l'agent d'ajustement de tension superficielle représente 0,01 % à 3 % en poids/volume de la composition.

13. Composition liquide suivant l'une quelconque des revendications précédentes, dans laquelle l'agent d'ajustement de viscosité est un polymère.

14. Composition liquide suivant l'une quelconque des revendications précédentes, dans laquelle l'agent d'ajustement de viscosité est un polyéthylèneglycol.

15. Composition liquide suivant la revendication 14, dans laquelle le polyéthylèneglycol a un poids moléculaire de 1 à 20 kilodaltons.

16. Composition liquide suivant l'une quelconque des revendications précédentes, dans laquelle l'agent d'ajustement de viscosité donne une solution ayant une viscosité de 2 cp à 10 cp.

17. Composition liquide suivant l'une quelconque des revendications précédentes, dans laquelle le constituant comprenant l'agent d'ajustement de viscosité et l'agent hydratant est un PEG.

18. Composition liquide suivant l'une quelconque des revendications précédentes, ayant une masse volumique de 0,7 g/ml à 2,2 g/ml.

19. Réservoir de fluide comprenant un réservoir contenant une composition liquide suivant l'une quelconque des revendications précédentes.

20. Utilisation d'un dispositif thermique d'éjection de gouttelettes pour produire un aérosol à partir d'une composition liquide suivant l'une quelconque des revendications 1 à 18.

21. Dispositif pour l'administration pulmonaire d'une protéine thérapeutique à un patient, dispositif comprenant un système électronique engendrant un aérosol, commandé par ordinateur, connecté par un fluide à un réservoir contenant une composition pharmaceutique liquide comprenant une protéine thérapeutique, un agent d'ajustement de tension superficielle et un agent hydratant, dans lequel la composition a une tension superficielle de 8 dynes/cm à 75 dynes/cm.

22. Dispositif suivant la revendication 21, dans lequel l'agent hydratant comprend un agent d'ajustement de viscosité.

23. Procédé pour la production d'un dispositif pour l'administration pulmonaire d'une protéine thérapeutique à un patient, procédé comprenant la connexion par un fluide d'un système électronique de production d'aérosol, commandé par ordinateur, à un réservoir contenant une composition pharmaceutique liquide comprenant une protéine thérapeutique, un agent d'ajustement de tension superficielle et un agent hydratant, et dans lequel la composition a une tension superficielle de 8 dynes/cm à 75 dynes/cm.

24. Procédé suivant la revendication 23, dans lequel l'agent hydratant comprend un agent d'ajustement de viscosité.
